# EUROPÄISCHE PATENTSCHRIFT

(11) **EP 2 516 525 B1**
(45) Veröffentlichungstag und Bekanntmachung des Hinweises auf die Patenterteilung: **18.05.2016**
(21) Anmeldenummer: 10790741.2
(22) Anmeldetag: 23.11.2010
(51) Int. Cl.: C08J 9/12, C08G 18/42, A61L 27/56, B01J 3/00, B29C 44/00

(54) **SCHAUMSTOFFFORMKÖRPER MIT ANISOTROPEN FORMGEDÄCHTNISEIGENSCHAFTEN, VERFAHREN ZU SEINER HERSTELLUNG UND ARTIKEL UMFASSEND DEN SCHAUMSTOFFFORMKÖRPER**
MOLDED FOAM BODY HAVING ANISOTROPIC SHAPE MEMORY PROPERTIES, METHOD FOR MANUFACTURING SAME AND ARTICLE COMPRISING THE MOLDED FOAM BODY
CORPS MOULÉ EN MOUSSE AYANT DES PROPRIÉTÉS ANISOTROPES DE MÉMOIRE DE FORME, PROCÉDÉ DE FABRICATION ASSOCIÉ ET ARTICLE COMPRENANT LE CORPS MOULÉ EN MOUSSE

(30) Priorität: 22.12.2009 DE 102009060940
(43) Veröffentlichungstag der Anmeldung: 31.10.2012
(73) Patentinhaber: Helmholtz-Zentrum Geesthacht Zentrum für Material- und Küstenforschung GmbH, 21502 Geesthacht (DE)
(72) Erfinder: LENDLEIN, Andreas, 14167 Berlin (DE); MADBOULY, Dr. Samy, 12613 Cairo (EG); KLEIN, Frank Andreas, 14089 Berlin (DE); KRATZ, Karl, 14615 Berlin (DE); LÜTZOW, Karola, 14195 Berlin (DE)
(74) Vertreter: Gulde & Partner
(86) Internationale Anmeldenummer: PCT/EP2010/068005
(87) Internationale Veröffentlichungsnummer: WO 2011/085847

(56) Entgegenhaltungen:
- EP-A1- 1 362 872
- WO-A1-2009/134425
- METCALFE A ET AL: "Cold hibernated elastic memory foams for endovascular interventions", BIOMATERIALS, ELSEVIER SCIENCE PUBLISHERS BV., BARKING, GB, Bd. 24, Nr. 3, 1. Februar 2003 (2003-02-01), Seiten 491-497, XP004390669, ISSN: 0142-9612, DOI: DOI:10.1016/S0142-9612(02)00362-9 in der Anmeldung erwähnt

## Beschreibung

Die Erfindung betrifft einen Schaumstoffformkörper mit anisotropen Formgedächtniseigenschaften, ein Verfahren zur Herstellung eines solchen Schaumstoffformkörpers sowie einen Artikel, der einen solchen Schaumstoffformkörper enthält oder aus diesem besteht.

Im Stand der Technik sind so genannte Formgedächtnispolymere oder SMPs (shape memory polymers) bekannt, die bei Induktion durch einen geeigneten Stimulus einen Formübergang von einer temporären Form in eine permanente Form entsprechend einer vorherigen Programmierung zeigen. Am häufigsten ist dieser Formgedächtniseffekt thermisch stimuliert, das heißt, bei Erwärmung des Polymermaterials über die definierte Schalttemperatur findet die durch Entropieelastizität angetriebene Rückstellung statt. Formgedächtnispolymere sind auf molekularer Ebene in der Regel Polymernetzwerke, bei denen chemische (kovalente) oder physikalische (nicht kovalente) Vernetzungsstellen die permanente Form bestimmen. Bei thermoplastischen Elastomeren sind die Netzpunkte physikalischer Natur. Neben den Netzpunkten sind die Formgedächtnispolymere aus Schaltsegmenten aufgebaut, welche die programmierte(n), temporäre(n) Form(en) fixieren. Dabei wird der temperaturinduzierte Übergang von einer programmierten temporären Form in die permanente Form oder einer weiteren temporären Form durch einen Phasenübergang des Schaltsegments entweder über eine Kristallisationstemperatur Tₘ oder eine Glasübergangstemperatur T_{g} angetrieben.

Eine Formgedächtnisfunktionalität wird durch die Kombination der oben genannten molekularen Struktur des Polymers sowie einer thermomechanischen Konditionierung (auch als Programmierung bezeichnet) erhalten. In einem ersten, klassischen Verarbeitungsschritt wird dabei die permanente Form des Polymers, beispielsweise im Wege des Spritzgießens oder dergleichen, hergestellt. Anschließend erfolgt die Programmierung, d.h. die Herstellung der temporären Form. Typischerweise wird hierfür das Polymer zunächst auf eine Temperatur oberhalb der Übergangstemperatur Tₜᵣₐₙₛ einer von dem Schaltsegment gebildeten Phase (= Schaltphase) erwärmt und bei dieser Temperatur das Polymermaterial in die temporäre Form deformiert. Unter Aufrechterhaltung der Deformationskräfte wird dann das Polymer unter die Übergangstemperatur abgekühlt, um durch den dabei hervorgerufenen Phasenübergang der Schaltphase, beispielsweise einer Kristallisation, die temporäre Form zu fixieren. Erneute Erwärmung oberhalb der Schalttemperatur führt zu einem Phasenübergang in umgekehrter Richtung, beispielsweise Schmelzen der Schaltphase, und Wiederherstellung der ursprünglichen permanenten Form. (Da die Schalttemperatur T_{switch} im Gegensatz zur rein materialabhängigen thermodynamischen Übergangstemperatur Tₜᵣₐₙₛ (Tₜᵣₐₙₛ = Tₘ oder T_{g}) von der zu leistenden Arbeit und der mechanischen Bewegung abhängt, welche die makroskopische Formveränderung definiert, können beide Temperaturen geringfügig voneinander abweichen.) Die Widerherstellung kann entweder spannungsfrei oder unter einer vorgegebenen Last erfolgen. Im Falle der spannungsfreien Wiederherstellung ergibt sich die charakteristische Schalttemperatur (T_{switch}) aus dem Wendepunkt der im Wesentlichen sigmoidal verlaufenden Dehnungs-Temperatur-Kurve. Im Falle der Rückstellung unter Last weist die Spannungs-Temperatur-Kurve ein charakteristisches Maximum auf, das die Schalttemperatur (T_{σ,max}) definiert. T_{σ,max} resultiert aus zwei gegensätzlichen bei der Erwärmung stattfindenden Prozessen, nämlich zum einen aus dem Anstieg der Kraft und zum anderen aus der zunehmenden Erweichung des Kunststoffs mit steigender Temperatur. Neben der oben beschriebenen typischen Programmierung oberhalb von Tₜᵣₐₙₛ wird im Stand der Technik weiterhin das Programmierungsverfahren der Kaltverstreckung (cold drawing) unterhalb von Tₜᵣₐₙₛ oder in der Nähe von Tₜᵣₐₙₛ beschrieben.

Darüber hinaus ist bekannt, poröse Formkörper (nachfolgend auch Schäume genannt) aus Formgedächtnispolymeren herzustellen (z.B. A. Metcalfe et al., Biomaterials 24 (2003), 491-497). Solche Formgedächtnisschäume besitzen ebenfalls die Fähigkeit, nach entsprechender thermomechanischer Programmierung (s.o.) eine komprimierte temporär fixierte Form einzunehmen und nach Erwärmung oberhalb der Schalttemperatur wieder in die ursprüngliche, permanente Gestalt zurückzukehren, d.h. zu expandieren. Formgedächtnisschäume, welche einen thermisch induzierten Formgedächtniseffekt zeigen, bestehen zumeist aus thermoplastischen Elastomeren (z.B. Multiblockcopolymeren, Polyesterurethanen), Blends (Polymermischungen) oder Kompositen (Polymer mit einem organischen oder anorganischen Füllstoff oder Additiv) der vorgenannten Kunststoffklassen. Die bekannten Formgedächtnisschäume sind zum Teil biokompatibel, jedoch sind keine resorbierbaren Schäume bekannt.

Nachteil der bisher im Stand der Technik beschriebenen Formgedächtnisschäume ist, dass sie bei Erwärmung eine in alle Raumrichtungen einheitliche (isotrope) Expansion zeigen. Für viele Anwendungen wünschenswert wäre jedoch ein anisotropes Ausdehnungsverhalten. Werden beispielsweise die bekannten isotrop komprimierten SMP-Schäume zum Ausfüllen komplexer Hohlräume insbesondere im medizinischen Bereich eingesetzt, wie dies etwa zum Füllen von Röhrenknochen beschrieben wurde (L.M. Mathieu et al., Biomaterials 27(6), (2006), 905-916), so kann das zu einer unvollständigen Verfüllung des Hohlraums oder zu unerwünschten mechanischen Verformungen führen.

EP 1362872 A1 offenbart ein Polyesterurethan mit Form-Gedächtnis-Eigenschaften und ein Verfahren zur Herstellung eines Schaums, wobei das Polyesterurethan in Gegenwart eines Treibmittels extrudiert und granuliert wird. Durch eine thermomechanische Programmierung wird es von einer temporären komprimierten Form zu einer permanenten isotropen Form expandiert.

WO 2009/134425 A1 offenbart ein Verfahren zur Herstellung eines Schaumstoffformkörpers mit anisotropen Poren. Die Poren werden durch die Entmischung und die Freisetzung des im Polymer gelösten C0₂im Wesentlichen entlang einer Vorzugsrichtung und somit zur Aufschäumung des Polymermaterials ausgebildet. Das Polymermaterial besteht aus reaktiven Oligomeren von Styrol oder Urethan.

Der vorliegenden Erfindung liegt daher die Aufgabe zugrunde, einen Schaumstoffformkörper mit Formgedächtniseigenschaften zur Verfügung zu stellen, der bei Erwärmung unterschiedlich starke Rückstellungen in unterschiedliche Raumrichtungen ausführen kann bzw. unterschiedliche Rückstellungskräfte ausübt. Zudem soll ein Verfahren vorgeschlagen werden, mit dem solche anisotropen Formgedächtnisschäume hergestellt werden können.

Diese Aufgabe wird durch einen Schaumstoffformkörper mit den Merkmalen des Anspruchs 1 gelöst. Der erfindungsgemäße Schaumstoffformkörper weist anisotrope, thermisch induzierbare Formgedächtniseigenschaften auf. Dabei wird im Rahmen der Erfindung unter dem Begriff "anisotrope Formgedächtniseigenschaften" ein Rückstellungsverhalten eines programmierten Schaumstoffformkörpers verstanden, das in unterschiedlichen Raumrichtungen mit unterschiedlichem Ausmaß stattfindet. Insbesondere bedeutet dies, dass ein komprimierter Schaumstoffformkörper bei Erwärmung eine unterschiedliche Expansion in die unterschiedlichen Raumrichtungen mit unterschiedlichen, richtungsabhängigen Expansionskräften zeigt. Dieses Verhalten trifft auch und gerade für die Rückstellung nach isotroper (richtungsunabhängiger) Programmierung zu. Der erfindungsgemäße Schaumstoffformkörper umfasst zumindest ein Formgedächtnispolymer, welches nach einer geeigneten thermomechanischen Programmierung oder mechanischen Programmierung (Kaltverstreckung) in der Lage ist, temperaturinduziert zumindest einen Formenübergang von einer temporären Form in eine permanente Form zu vollziehen. Dabei zeichnet sich der erfindungsgemäße Formgedächtnisschaumstoff dadurch aus, dass er eine Schaumstruktur mit asymmetrischen, im Wesentlichen in einer gemeinsamen, ersten Raumrichtung (longitudinale Raumrichtung L) ausgerichteten Poren aufweist. Es wurde nämlich überraschend gefunden, dass sofern es gelingt, einen Formgedächtnispolymerschaum mit einer solchen asymmetrischen, gleichgerichteten Porenstruktur herzustellen, dieser die Eigenschaft aufweist, nach einer entsprechenden Programmierung ein unterschiedliches Maß an Ausdehnung in die verschiedenen Raumrichtungen auszuführen beziehungsweise die Rückstellung mit unterschiedlichen, richtungsabhängigen Expansionskräften in die verschiedenen Raumrichtungen zu vollziehen.

Dabei kann der Grad an Anisotropie gezielt über die Gestaltung der Schaummorphologie beziehungsweise Porenstruktur eingestellt werden. Dabei wird unter einer asymmetrischen Porenstruktur verstanden, dass ein Verhältnis einer mittleren Porendimension in der ersten (longitudinalen) Raumrichtung zu einer mittleren Porendimension in einer zweiten, orthogonal zu dieser verlaufenden (transversalen) Raumrichtung ungleich 1 ist. Insbesondere beträgt das Verhältnis der Porendimension in der longitudinalen zu der transversalen Raumrichtung zumindest 2, das heißt der mittlere longitudinale Porendurchmesser ist zumindest doppelt so groß wie der transversale Porendurchmesser. In vorteilhaften Ausgestaltungen beträgt das Verhältnis von longitudinaler zu transversaler Richtung zumindest 3, insbesondere zumindest 4 und besonders bevorzugt zumindest 5. Dabei zeigt sich, dass je größer dieses Verhältnis ist, desto ausgeprägter die Anisotropie des Formgedächtniseffekts ist.

Vorzugsweise weist der erfindungsgemäße Schaum eine Dichte des Schaumstoffformkörpers im Bereich von 0,01 bis 0,30 g/cm³ auf, insbesondere von 0,02 bis 0,20 g/cm³, besonders bevorzugt von 0,07 bis 0,13 g/cm³. Dabei ist die untere Grenze dadurch limitiert, dass der Polymerschaum eine gewisse Rückstellkraft (Expansionskraft) aufweisen muss, um seine bestimmungsgemäße Funktion zu erfüllen. Auf der anderen Seite ist bei Schaumstoffmaterialen grundsätzlich eine "biokompatible" Oberflächenstruktur sowie ein geringes Gewicht erwünscht, was die obere Grenze limitiert.

Auf molekularer Ebene ist das Formgedächtnispolymer vorzugsweise ein Polymernetzwerk, das über physikalische (nicht-kovalente) Vernetzungsstellen verfügt sowie über Schaltsegmente, die eine Übergangstemperatur Tₜᵣₐₙₛ (T_{g} oder Tₘ) in einem für die jeweilige Anwendung akzeptablen Bereich aufweisen. Beispielsweise sollte die Übergangstemperatur des Schaltsegments bei physiologischen Anwendungen in einem Bereich von 10 bis 80°C liegen. Vorzugsweise handelt es sich bei dem Formgedächtnispolymer um ein physikalisch vernetztes thermoplastisches Elastomer. Insbesondere kann das Schaltsegment ausgewählt sein aus der Gruppe der Polyester, insbesondere Poly(ε-caprolacton); Polyether, Polyurethane, insbesondere Polyurethan; Polyimide, Polyetherimide, Polyacrylate, Polymethacrylate, Polyvinyle, Polystyrole, Polyoxymethyle, Poly(para-dioxanon). Weiterhin ist bevorzugt, dass das Formgedächtnispolymer hydrolytisch spaltbare Gruppen aufweist, beispielsweise Glycolid- oder Lactid-Gruppen. Insbesondere kann es sich hier um Diclycolid-Gruppen, Dilactid-Gruppen, Polyanhydriden oder Polyorthoester handeln. Sofern ein geeigneter Anteil hydrolytisch spaltbarer Gruppen im Polymer vorhanden ist, ist der erfindungsgemäße Schaumstoff resorbierbar (abbaubar). Dabei kann die hydrolytische Abbaurate über den Anteil der hydrolytisch spaltbaren Gruppen in weiten Bereichen eingestellt werden.

Vorzugsweise ist das Polymer ein Copolymer mit mindestens einem Hart- und mindestens einem Weichsegment, wobei Hart- und Weichsegmente jeweils zumindest eine Übergangstemperatur wie Glasübergänge, Phasenumwandlungen, Schmelztemperaturen oder Schmelzintervalle aufweisen, die einen ausreichenden Abstand zueinander aufweisen, der eine Programmierung und anschließende Rückstellung erlaubt. Insbesondere sollten die Übergangstemperaturen des Weichsegments und des Hartsegments um mindestens 1 K, insbesondere mindestens 10 K, vorzugsweise mindestens 50 K und in besonderen Fällen um mindestens 200 K auseinander liegen. Basierend auf den vorstehend beschriebenen Polymeren liegen die Übergangstemperaturen (hier Glasübergangstemperaturen) im Bereich von -60°C bis +250°C.

Ein weiterer Aspekt der Erfindung betrifft einen Artikel, der aus dem erfindungsgemäßen Schaumstoffformkörper (vollständig) besteht oder einen solchen (beispielsweise abschnittsweise) enthält. Dabei kann es sich insbesondere um einen Gegenstand zur Anwendung im medizinischen, pharmazeutischen oder biochemischen Bereich handeln. Beispielsweise kann der Artikel ein pharmazeutisches Produkt sein, das den erfindungsgemäßen Schaumstoffformkörper als Trägersubstanz für einen pharmazeutischen Wirkstoff, mit dem es beladen ist, enthält. Sofern es sich gleichzeitig bei dem Formgedächtnispolymer um ein abbaubares Polymer handelt (siehe oben), wird der Wirkstoff retardiert freigesetzt. Weitere interessante Anwendungen betreffen den Einsatz als Medizinprodukt im Bereich des so genanten Tissue Engeneerings im Allgemeinen, bei dem der Artikel, als Träger zur Besiedlung mit Zellen eingesetzt wird, als Strukturkörper für den Hart- oder Weichgewebeersatz, als mechanisch aktiver Scaffold oder dergleichen.

Die Erfindung betrifft zudem ein Verfahren zur Herstellung des erfindungsgemäßen Schaumstoffformkörpers mit anisotropen, thermisch induzierbaren Formgedächtniseigenschaften. Das Verfahren umfasst die Schritte:
(a) Herstellen einer im Wesentlichen homogenen Mischung oder Lösung umfassend eine Schmelze eines Formgedächtnispolymers (welches nach einer Programmierung in der Lage ist, temperaturinduziert zumindest einen Formenübergang von einer temporären Form in eine permanente Form zu vollziehen) und eines in einem ersten Zustand vorliegenden Treibmittels oder einer chemischen Vorstufe eines solchen,
(b) Überführen und Freisetzen des Treibmittels in einen zweiten, gasförmigen Zustand derart, dass das Formgedächtnispolymer und das Treibmittel entmischen, wobei Prozessparameter der Freisetzung und eine Geometrie eines Prozessbehälters, in welchem die Freisetzung des Treibmittels erfolgt, so gewählt sind, dass das freigesetzte gasförmige Treibmittel im Wesentlichen entlang einer vorbestimmten Hauptströmungsrichtung aus dem Formgedächtnispolymer entweicht, so dass das Formgedächtnispolymer eine Schaumstruktur mit asymmetrischen, im Wesentlichen in einer gemeinsamen ersten, der Hauptströmungsrichtung entsprechenden longitudinalen Raumrichtung (L) ausgerichteten Poren ausbildet.

Erfindungsgemäß werden somit während des Verschäumungsprozesses Bedingungen geschaffen, die dafür sorgen, dass das freigesetzte Treibmittel im Wesentlichen entlang einer Vorzugsrichtung (der Hauptströmungsrichtung) aus dem Polymermaterial entweicht. Dies führt dazu, dass das Polymermaterial asymmetrische Poren ausbildet, die entsprechend dieser Hauptströmungsrichtung (nachfolgend auch longitudinale Richtung L genannt) ausgerichtet sind.

Es versteht sich, dass nach dem Verschäumen oder währenddessen eine Absenkung der Temperatur unterhalb der Schmelztemperatur der Schmelze unter Verfestigung (Fixierung) der Porenstruktur erfolgt. Dabei kann entweder eine Kühlung unter aktivem Wärmeentzug erfolgen und/oder eine passive Kühlung im Wege eines endothermen Verschäumungsprozesses.

Als Treibmittel kann entweder ein chemisches oder ein physikalisches Treibmittel eingesetzt werden. Dabei bezeichnet man als chemische Treibmittel solche, die gezielt zu einer chemischen Aufspaltung (beispielsweise im Wege der Bestrahlung) angeregt werden können, wobei sich das eigentliche Treibmittel in gasförmigem Zustand (beispielsweise Stickstoff N₂) bildet. In diesem Falle wird also eine chemische Vorstufe des eigentlichen Treibmittels verwendet. Vorzugsweise wird im Rahmen der vorliegenden Erfindung jedoch ein physikalisches Treibmittel verwendet, das während des Herstellungsverfahrens keine chemische Reaktion durchläuft. Vielmehr liegt das physikalische Treibmittel unter der im Schritt a) angewandten Temperatur und dem angewandtem Druck in einem flüssigen, festen oder superkritischen Zustand vor. In diesem Fall erfolgt die Freisetzung des Treibmittels in Schritt b) dadurch, dass die Temperatur und/oder der Druck so verändert wird bzw. werden, dass das Treibmittel in den gasförmigen Zustand übergeht und somit zu einer Separierung vom Polymer und zur Ausbildung der Poren führt. Das expandierende Treibmittel entzieht gleichzeitig seiner Umgebung Energie in Form von Wärme, wodurch eine Abkühlung des Polymers unterhalb der Schmelztemperatur eintritt und der entstandene Schaum stabilisiert wird. Vorzugsweise wird im Rahmen der vorliegenden Erfindung Kohlendioxid als physikalisches Treibmittel verwendet, das in Schritt a) in superkritischem Zustand vorliegt und insbesondere durch eine Druckabsenkung in Schritt b) in den gasförmigen Zustand übergeht. Superkritisches Kohlendioxid weist in den meisten Polymermaterialien eine sehr hohe Löslichkeit auf. Der Verzicht von chemischen Treibmitteln sowie von anorganischen oder organischen Lösungsmitteln ist besonders dann von Vorteil, wenn die resultierenden Schäume für den Einsatz als Medizinprodukt oder als temporäres Implantat bestimmt sind.

Wie oben bereits bemerkt wurde, wird die Freisetzung des Treibmittels in einer Hauptströmungsrichtung einerseits über die Prozessparameter bei der Freisetzung verursacht und andererseits über die Wahl der Geometrie des Prozessbehälters, in welchem die Aufschäumung erfolgt. Vorzugsweise ist der Prozessbehälter so gewählt, dass dieser in die longitudinale Raumrichtung (also der gewünschten Hauptströmungsrichtung des Treibmittels) ein- oder beidseitig geöffnet ist und in die anderen Raumrichtungen geschlossen. Auf diese Weise werden sämtliche Strömungsrichtungen außer der longitudinalen gesperrt, so dass das Treibmittel im Wesentlichen in die longitudinale Richtung entweicht. Eine weitere die Geometrie des Prozessbehälters betreffende Maßnahme stellt die Wahl seiner Dimensionen dar. Insbesondere ist sein Höhe-Breiten-Verhältnis so gewählt, dass die Mischung aus Polymer und Treibmittel eine Säule im Prozessbehälter einnimmt, die in der longitudinalen Richtung eine größere Ausdehnung aufweist als in der transversalen Richtung. Mit anderen Worten, weist ein geeigneter Prozessbehälter (bei entsprechender Füllung) eine längliche Gestalt auf, wobei seine Breite beziehungsweise sein Durchmesser durch seine Höhe deutlich überstiegen wird.

Prozessparameter während des Verschäumungsprozesses, welche die Porenmorphologie und insbesondere ihre Asymmetrie beeinflussen, umfassen insbesondere die Verschäumungstemperatur, den Verschäumungsdruck, den Anteil des eingesetzten Treibmittels bezogen auf das Formgedächtnispolymer sowie die Geschwindigkeit, mit der das Treibmittel in Schritt b) freigesetzt wird. Im Falle der Verwendung eines physikalischen Treibmittels, insbesondere von superkritischem Kohlendioxid, handelt sich somit um die Verschäumungstemperatur, den Verschäumungsdruck, den Sättigungsgrad des Polymers mit dem superkritischen Kohlendioxid (beziehungsweise der Dauer der Beaufschlagung des Polymers mit dem superkritischen Kohlendioxid) sowie um die Entspannungsrate, mit der in Schritt b) der Druck abgesenkt wird. Dabei gilt, dass je höher die Entspannungsrate ist, desto stärker die Asymmetrie der Poren und damit der Anisotropie des Formgedächtniseffekts. Insbesondere wird eine Druckabsenkungsrate von 0,1 bar/s bis 100 bar/s, vorzugsweise von mindestens 10 bar/s, besonders bevorzugt von mindestens 15 bar/s und ganz besonders bevorzugt von mindestens 20 bar/s eingestellt.

Sofern - wie oben bereits beschrieben wurde - der Schaum mit einem pharmazeutischen Wirkstoff ausgestattet werden soll, so lässt sich dies besonders einfach und schonend realisieren, wenn der Wirkstoff in dem flüssigen oder superkritischen Treibmittel, beispielsweise in dem superkritischen Kohlendioxid, gelöst wird und das Polymer in Schritt (a) mit diesem Gemisch beaufschlagt wird. Es versteht sich, dass auch andere Substanzen als pharmazeutische Wirkstoffe auf diese Weise eingebracht werden können. Im Ergebnis liegt die Substanz nach der Entspannung als Feststoff oder Flüssigkeit fein dispergiert im Schaum vor.

Die thermomechanische Programmierung des Schaumstoffformkörpers erfolgt in einem mittelbar oder unmittelbar an die Aufschäumung in Schritt b) anschließenden in Schritt c) vorzugsweise dadurch, dass bei einer Materialtemperatur oberhalb der Schalttemperatur des Formgedächtnispolymers beziehungsweise des Schaltsegments der Schaumstoffformkörper entsprechend einer gewünschten temporären Form deformiert (insbesondere komprimiert) wird und unter Aufrechterhaltung des Formenzwangs auf eine Temperatur unterhalb der Schalttemperatur abgekühlt wird, wobei die durch die Deformation aufgezwungene temporäre Form fixiert wird. Die Wiederherstellung der permanenten (expandierten) Form erfolgt durch Erwärmung des Materials auf eine Temperatur oberhalb der Schalttemperatur des Materials. Alternativ kann die Programmierung wie eingangs bereits erläutert auch durch so genannte Kaltverstreckung erfolgen, wobei die Deformation bei Temperaturen unterhalb oder in der Nähe der Schalttemperatur erfolgt.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen erläutert. Es zeigt:
- Figur 1: schematisch ein Beispiel für eine Schäumungsvorrichtung zur Herstellung des erfindungsgemäßen Schaumstoffes mit asymmetrischer Porenstruktur;
- Figuren 2 a)-b): Beispiele für Prozessgefäße zur Benutzung in einer Vorrichtung gemäß Figur 1;
- Figur 3: zeitliche Verläufe der Prozessparameter Druck und Probentemperatur während des Verschäumungsprozesses;
- Figur 4 a)-c): Versuchsaufbau zur Erzeugung eines anisotropen SMP-Schaumstoffs (a) sowie EM-Aufnahmen eines Schnittes durch einen so erzeugten Schaumstoff in longitudinaler (b) und transversaler (c) Ebene;

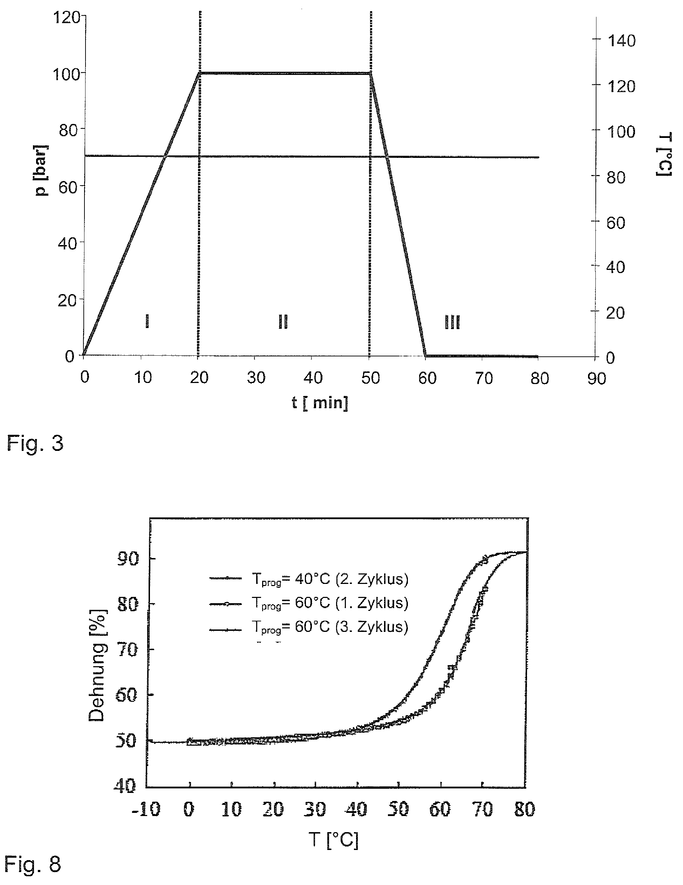

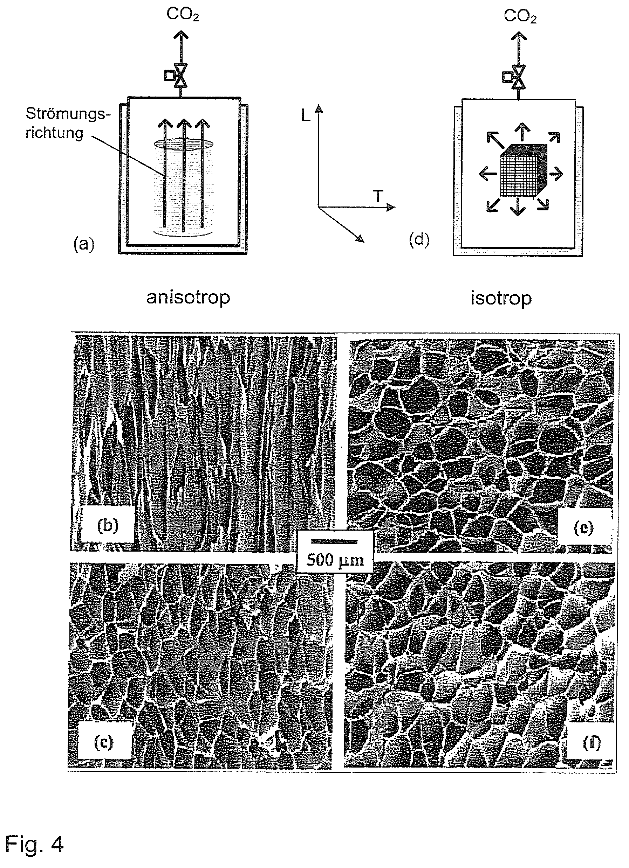

- Figur 4 d)-f): Versuchsaufbau zur Erzeugung eines isotropen SMP-Schaumstoffs (d) sowie EM-Aufnahmen eines Schnittes durch einen so erzeugten Schaumstoff in longitudinaler (b) und transversaler (c) Ebene;

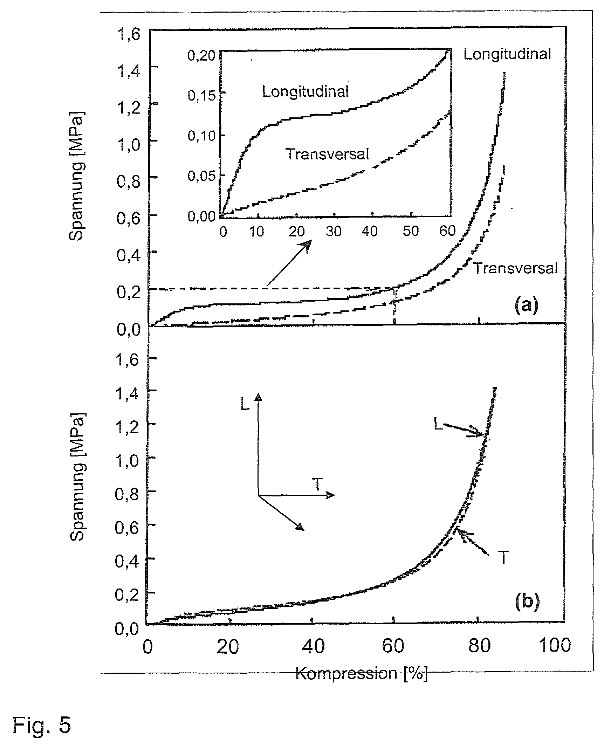

- Figur 5: mechanische Eigenschaften eines nicht programmierten anisotropen (a) und isotropen (b) SMP-Schaumstoffs im Druckversuch;

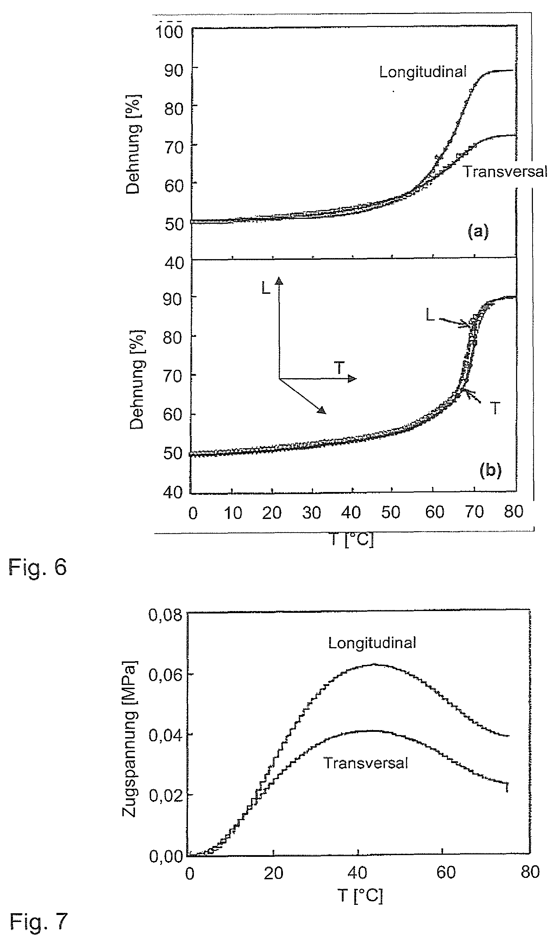

- Figur 6: spannungsfreies temperaturinduziertes Rückstellverhalten eines programmierten anisotropen (a) und isotropen (b) SMP-Schaumstoffs;
- Figur 7: temperaturinduziertes Rückstellverhalten eines programmierten anisotropen (a) SMP-Schaumstoffs unter Last, und
- Figur 8: spannungsfreies temperaturinduziertes Rückstellverhalten eines bei zwei unterschiedlichen Programmierungstemperaturen programmierten anisotropen SMP-Schaumstoffs.

Bei dem Formgedächtnispolymer handelt es sich um ein Polymernetzwerk mit thermisch induzierbarem Formgedächtniseffekt (SMP). Die Netzwerkbildung kann dabei durch kovalente Bindungen realisiert sein oder durch physikalische Wechselwirkungen, beispielsweise elektrostatische Effekte. Neben den Vernetzungspunkten umfasst das Polymernetzwerk zumindest eine Sorte eines Schaltsegments, das eine materialabhängige Übergangstemperatur, etwa eine Kristallisationstemperatur oder Glasübergangstemperatur aufweist. Polymernetzwerke, die einen Formgedächtniseffekt aufweisen, sind in der Literatur zahlreich beschrieben. Grundsätzlich ist die vorliegende Erfindung auf kein spezielles Material beschränkt. Beispielsweise kann das Polymernetzwerk ein Schaltsegment aufweisen, das ausgewählt ist aus der Gruppe der Polyester, insbesondere Poly(ε-caprolacton); Polyether, Polyurethane, insbesondere Polyurethan; Polyimide, Polyetherimide, Polyacrylate, Polymethacrylate, Polyvinyle, Polystyrole, Polyoxymethyle, Poly(para-dioxanon) oder andere. Denkbar ist ebenfalls, dass das Polymernetzwerk zwei oder mehr unterschiedliche Schaltsegmente aus der vorstehenden Gruppe oder andere aufweist. Dabei wird das zumindest eine Schaltsegment vorzugsweise so gewählt, dass seine Schalttemperatur in einem für die jeweilige Anwendung akzeptablen Bereich liegt. Optional kann das Formgedächtnispolymer hydrolytisch spaltbare Gruppen aufweisen, insbesondere Diglycolide, Dilactide, Polyanhydride oder Polyorthoester. Auf diese Weise werden bioabbaubare Materialien erhalten, was insbesondere für Anwendungen im biomedizinischen Bereich vorteilhaft sein kann. Auch bioabbaubare Formgedächtnispolymere sind aus der Literatur hinlänglich bekannt. Die vorliegende Erfindung ist auf keine speziellen Vertreter dieser Gruppe eingeschränkt.

Die vorliegende Erfindung betrifft ein Schaummaterial, das aus einem solchen Formgedächtnispolymer hergestellt ist und das eine asymmetrische Porenmorphologie mit im Wesentlichen gleich ausgerichteten Poren aufweist, was dem Schaum ein anisotropes Formgedächtnisverhalten verleiht. Die Morphologie wird dabei durch die Wahl der Prozessparameter und/oder der Eigenschaften des Prozessbehälters, in dem die Verschäumung durchgeführt wird, gezielt beeinflusst.

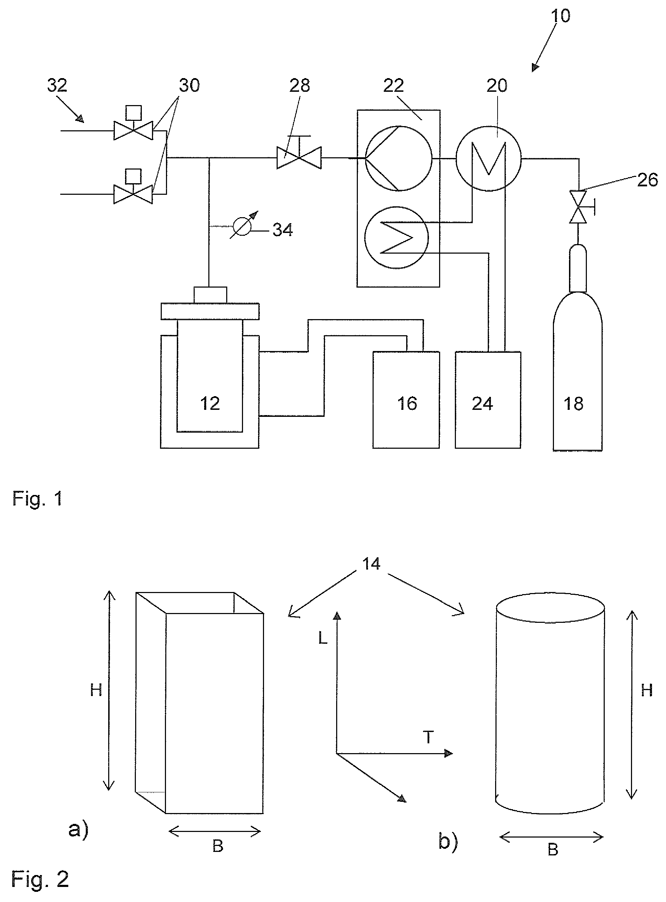

Figur 1 zeigt schematisch eine Vorrichtung 10 zur Durchführung des erfindungsgemäßen Schäumungsprozesses. Die Vorrichtung 10 umfasst einen Druckbehälter 12 zur Aufnahme eines in Figur 2 gezeigten Prozessbehälters 14 mit der aufzuschäumenden Probe. Der Druckbehälter 12 weist einen isolierenden Doppelmantel zur Temperierung auf, der mit einem Thermostaten 16 über Kühlmittel führende Leitungen verbunden ist.

Ein im Druckbehälter 12 befindlicher Probenraum ist über ein Leitungssystem mit einem Treibmittelvorratsbehälter 18, der insbesondere CO₂ enthält, verbunden. Die Temperierung und Druckeinstellung des den Druckbehälter 12 beaufschlagenden Treibmittels erfolgt mittels eines Wärmetauschers 20 beziehungsweise eines kombinierten Pumpe-Wärmetauscher-Moduls 22. Der Wärmetauscher 20 und der Wärmetauscher des Pumpe-Wärmetauscher-Moduls 22 sind über Kühlmittel führende Leitungen mit einem Krystaten 24 verbunden Die Treibmittelzuführung kann mittels jeweils eines saug- bzw. druckseitig angeordneten Absperrorgans 26, 28 unterbrochen werden.

Über ein, zwei Belüftungsventile 30 aufweisendes Entspannungsmodul 32, das mit einem Drucktransmitter 34 zur Messung und Übermittlung des Drucks im Druckbehälter 12 beziehungsweise in der druckseitigen Leitung in Signalverbindung steht, erlaubt eine geregelte Belüftung des Druckbehälters 12.

Figuren 2 a und 2 b zeigen zwei Beispiele für Prozessbehälter 14 zur Aufnahme der aufzuschäumenden Probe, die im Rahmen der vorliegenden Erfindung Anwendung finden können. Es handelt sich jeweils um einseitig (nach oben) oder beidseitig (oben und unten) geöffnete Gefäße mit beispielsweise quadratischem oder rechteckigem (Figur 2a) beziehungsweise rundem (Figur 2b) Grundriss. Vorteilhaft ist jeweils, dass die Höhe H des Gefäßes größer als die größte Seitenlänge B beziehungsweise der Durchmesser B des Gefäßes ist. Bevorzugt wird die Geometrie des Prozessgefäßes so gewählt, dass H ≥ 1,5 B ist, d.h. dass die Höhe H mindestens dem Eineinhalbfachen der (längsten) Seitenlänge B bzw. des Durchmessers B ist. Vorzugsweise gilt H ≥ 2 B und besonders bevorzugt H ≥ 3 B. Durch Wahl des Höhe/Breite-Verhältnisses des Prozessgefäßes kann der Asymmetriegrad der erzeugten Poren mitbestimmt werden.

Die in Figur 1 gezeigte Schäumungsvorrichtung 10 zeigt folgende Funktionsweise. Eine aufzuschäumende Probe eines Formgedächtnispolymers wird in einen Prozessbehälter 14 beispielsweise gemäß Figur 2 a oder 2 b und dieser in den Druckbehälter 12 eingebracht und über den Thermostaten 16 auf eine vorbestimmte Prozesstemperatur temperiert. Nach dem Öffnen der saug- und druckseitigen Absperrorgane (Ventile) 26 und 28 beginnt der Füllvorgang des Druckbehälters 12. Das flüssige CO₂ strömt aus dem Vorratsbehälter 18 bis zum Druckgleichgewicht in den Druckbehälter 12. Um den Druck im Druckbehälter 12 auf den gewünschten Sättigungsdruck einzustellen, muss das durch Kühlen mit dem Wärmetauscher 20 flüssig gehaltene CO₂ durch das Pumpe-Wärmetauscher-Modul 22 komprimiert werden. Im Druckbehälter 12 erfolgt ab etwa 73,7 bar und oberhalb von 31°C der Übergang in den überkritischen Bereich. Dabei löst sich das scCO₂ im Formgedächtnispolymer, wobei eine einphasige Lösung entsteht. Die Beaufschlagung wird solange durchgeführt, bis sich eine vorbestimmte, von den Prozessparametern Temperatur und Druck abhängige Sättigung des Polymers mit dem CO₂ einstellt.

Sodann wird das Absperrorgan 26 und/oder 28 geschlossen und es erfolgt mit Hilfe des Entspannungsmoduls 32 eine geregelte Belüftung des Druckbehälters 12, wobei die Belüftungsventile 30 unter Erfassung des Drucks mittels des Drucktransmitters 34 so angesteuert werden, dass eine vorbestimmte, Belüftungsrate eingeregelt wird. Aufgrund des Druckabfalls kommt es zu einer Ausgasung des CO₂ aus dem Polymermaterial. Die zuvor einphasig vorliegende Lösung des scCO₂ in dem Polymer geht in ein Zweiphasensystem (festes Polymer/gasförmiges CO₂) über. Diese Phasenseparierung führt zu der gewünschten Aufschäumung des Polymers, während das gasförmige CO₂ entweicht.

Figur 3 stellt beispielhaft zeitliche Verläufe der Prozessparameter Druck und Probentemperatur während des Verschäumungsprozesses dar. Es lassen sich drei Phasen unterscheiden. In Phase I, die mit der Öffnung des Absperrorgans 28 und somit der Beaufschlagung des Druckbehälters 12 und der Polymerprobe mit dem CO₂ beginnt, erfolgt der Druckaufbau im Druckbehälter 12, bis dort beispielsweise ein Druck von etwa 100 bar erreicht ist. Zu diesem Zeitpunkt beginnt Phase II (Diffusionsphase), in welcher die Sättigung des Polymers mit dem Treibmittel stattfindet, währenddessen der Druck annähernd konstant bleibt und die Temperatur ihren vorgegebenen Sollwert erreicht. Ist das Gleichgewicht erreicht, beginnt Phase III mit der geregelten Druckentlastung, währenddessen der Druckabfall erzeugt wird und die Schaumformierung stattfindet. Durch die Temperaturabsenkung (Kühlung) wird die geschäumte Probe stabilisiert.

Durch die Verwendung eines einseitig oder beidseitig offenen, vorzugsweise länglichen Prozessbehälters gemäß Figur 2 wird ein gerichteter Gasfluss des während der Phase III (in Figur 3) entweichenden Treibmittels im Wesentlichen parallel zu den Seitenwänden des Behälters in die longitudinale Raumrichtung L erzielt (s. Koordinatendefinition in Figur 2). Infolgedessen entsteht eine asymmetrische Porenstruktur des erzeugten Schaums, bei der die Porenlänge deutlich größer als der Porendurchmesser ist. Zudem weisen die Poren eine im Wesentlichen gleichgerichtete Ausrichtung auf, nämlich entlang der Hauptströmungsrichtung des Treibmittels, d.h. parallel zu den Gefäßseitenwänden in die longitudinale Raumrichtung L. Durch Wahl des Prozessbehälters, nämlich seines Höhe/Breite-Verhältnisses und seiner Grundform, sowie durch die Prozessparameter Verschäumungstemperatur, Sättigungsgrad des Polymeres mit dem Treibmittel und die Entspannungsgeschwindigkeit (Belüftungsrate) kann die Porosität, die Porengröße, die Porengrößenverteilung, die Schaumdichte sowie das Ausmaß der Asymmetrie der Poren, d.h. das Verhältnis der Porenlänge zu dem Porendurchmesser beeinflusst werden. Eine besonders wichtige Rolle hinsichtlich Porengröße, Schaumdichte und mechanischer Komprimierbarkeit spielt dabei die Belüftungsrate. Ein große mittlere Porengröße (500 ±50 µm) mit einer hohen Dichte (0,3 ±0,05 g/cm³), geringer Komprimierbarkeit und hoher Steifigkeit wurden bei niedrigen Belüftungsraten (< 10 bar/s) erhalten. Hingegen wurden Schäume geringer Dichte (0,11 ±0,02 g/cm³), kleines Porengrößen (150-200 µm) und mit sehr guter und thermomechanischem Rückstellverhalten wurden für das getestete System bei Temperaturen von 78 bis 84°C bei hohen Belüftungsraten (25 bar/s) erzielt.

Nachfolgend wird das Prinzip der vorliegenden Erfindung anhand eines asymmetrischen Schaums aus einem Multiblockcopolymer (PDLCL), hergestellt durch Verknüpfung der Makrodiole von Poly(ω-pentadecalacton) PPDL und Poly(ε-caprolacton) PCL im Wege einer Cokondensation, beschrieben. In dem Polymer PDLCL fungiert das PPDL als kristallisierbares Hartsegment (Tₘ(PPDL) = 82°C) und PCL als ebenfalls kristallisierbares Weichsegment (Tₘ(PCL) = 48°C). Beide Segmente sind kovalent miteinander über Hexamethylendiisocyanat als Verbindungsglied verknüpft.

### 1. Herstellung des Multiblockcopolymers PDLCL aus Poly(ω-pentadecalacton) und Poly(ε-caprolacton)

Die beiden endständigen Makrodiole Poly(ω-pentadecalacton)-diol (PPDL-diol; *M*ₙ = 2200 g·mol⁻¹ and *T*ₘ = 89 °C, selbst synthetisiert) und Poly(ε-caprolacton)-diol (PCL-diol; *M*ₙ = 3200 g·mol⁻¹ and *T*ₘ = 44 °C, Solvay, UK) wurden mit Hexamethylendiisocyanat (HMDI; Fluka, Deutschland) und Dibutyltinlaurat in 1,2-Dichlorethan bei 80 °C umgesetzt. Das Produkt PDLCL wurde durch Fällung in Methanol bei -10°C erhalten. Details der Herstellung sind in K. Kratz, U. Voigt, W. Wagermaier, A. Lendlein: "Shape-memory properties of multiblock copolymers consisting of poly(ε-pentadecalactone) hard segments and crystallizable poly(ε-caprolactone) switching segments", in Advances in Material Design for Regenerative Medicine, Drug Delivery, and Targeting/Imaging, edited by V. Prasad Shastri, A. Lendlein, L-S. Liu, A. Mikos, S. Mitragotri (Mater. Res. Soc. Symp. Proc. Volume 1140, Warrendale, PA, 2009) und S. A. Madbouly, K. Kratz, F. Klein, K. Lützow, A. Lendlein: "Thermomechanical behaviour of biodegradable shape-memory polymer foams", in Active Polymers, edited by A. Lendlein, V. Prasad Shastri, K. Gall (Mater. Res. Soc. Symp. Proc. Volume 1190, Warrendale, PA, 2009) beschrieben.

Das PDLCL wies gemäß ¹H-NMR-Analyse (500 MHz Bruker Advance Spectrometer, Deutschland) einen Gewichtsanteil von 60 Gew.-% PCL und 40 Gew.-% PPDL auf (gemessen in deuteriertem Chloroform mit Tetramethylsilan als internem Standard).

Zur Berechnung des Molekulargewichts Mₙ und der Molekulargewichtsverteilung (Polydisperisität PD) wurden GPC-Messungen basierend auf konventionellen Kalibrierverfahren unter Verwendung von Polystyrol-Standards mit Mₙ zwischen 1.000 und 3.000.000 g/mol (Polymer Standards Service GmbH, Deutschland) durchgeführt. Das PDLCL wies ein Molekulargewicht Mₙ = 58.000 g/mol und eine Polydispersität von 2,2 auf.

### 2. Schäumungsprozess

Vor der Aufschäumung wurde das PDLCL-Rohmaterial bei 95°C in einem Einschneckenextruder (Thermo Electron I/d 34, d = 19 mm) granuliert.

Das Schäumungsverfahren wurde unter Verwendung einer Schäumungsvorrichtung nach Figur 1 gemäß dem vorstehend beschriebenen erfindungsgemäßen Verfahren durchgeführt. Hierzu wurde die Polymerprobe mit superkritischem Kohlendioxid (scCO₂ bei 79 °C und einem konstanten Druck von 200 bar bis zur Einstellung eines Sättigungsgleichgewichts für mindestens etwa 30 min beaufschlagt. Nach 60 min (inklusive des Füllvorgangs) wurde das Belüftungsprogramm gestartet, wobei die Belüftungsventile gesteuert (Imago 500) geöffnet wurden, sodass eine Druckentspannung mit einer Rate von 25 bar/s eingestellt wurde. Diese sehr schnelle Belüftungsrate führte zu einer Entmischung und Expansion des im Polymer gelösten CO₂ und somit zur Aufschäumung des Polymermaterials. Nach Erreichen des Umgebungsluftdrucks wurden die Schaumstoffproben aus dem Gefäß entnommen und unter Raumbedingungen für 24 h gelagert.

Zur Erzeugung der erfindungsgemäßen anisotropen Schäume wurden als Prozessbehälter 14 für die Aufschäumung kleine Bechergläser (29 mm Durchmesser, 50 mm Höhe) aus Glas verwendet, d.h. mit zylindrischer Form gemäß Figur 2 b. Während des plötzlichen Druckanstiegs floss das entweichende CO₂ entlang einer Hauptströmungsrichtung im Wesentlichen parallel zu den seitlichen Becherglaswänden. Dieser Aufbau ist in Figur 4 a skizziert, in der die vertikalen Pfeile die longitudinale Hauptströmungsrichtung L des austretenden CO₂ darstellen.

Als Vergleichsversuch wurden Proben auf die gleiche Weise aufgeschäumt, wobei jedoch anstelle des Becherglases ein allseitig gasdurchlässiges Gefäß aus einem geschlossenen Metallnetz mit einer Maschenweite im Mikrometerbereich Verwendung fand (Figur 4d). Mit gleichem Resultat kann auch ein Gefäß aus einer gasdurchlässigen Membran eingesetzt werden.) Dabei lag das Polymermaterial als Granulat vor. Unter diesen Bedingungen entwich während der schnellen Belüftung das CO₂ annähernd homogen in alle Raumrichtungen aus der aufschäumenden Probe und dem Gefäß (siehe Pfeile in Figur 4d).

### 3. Porosität und Schaummorphologie

Die Porengrößenverteilungen wurden durch Quecksilber-Porositätsmessungen ermittelt (Mercury Porosimeter Pascal 140 und 440, Fisons Instruments, Italien).

Die Porosität und der relative Anteile offener, einem Verdrängungsmedium zugänglichen Poren wurde mittels pycnometrischer Messungen nach der Anleitung des Geräteherstellers in einer 60 cm³ Testzelle bei 20°C (Ultrafoam Pycnometer 1000, Quantachrome Instruments, USA) unter Verwendung von Stickstoff als Verdrängungsmedium (6 psi) und 10-fachen Wiederholungsmessungen durchgeführt. Die absolute Porosität ergab sich aus dem Verhältnis des Volumens der geschäumten Probe zum Volumen der ungeschäumten Probe. Alle Proben wiesen ein hohes Maß an Porosität von etwa 89 ±4 % auf und einen Anteil offenzelliger Poren von etwa 41 ±4 % bezogen auf die Anzahl aller Poren. Die Schaumdichte wurde aus dem geometrischen Volumen und der Masse der Schaumprobe berechnet.

Zur Ermittlung der Porenstruktur der nach dem erfindungsgemäßen Verfahren und im Vergleichsversuch hergestellten Schäume wurden die aufgeschäumten Proben nach Lagerung für wenige Minuten in flüssigem Stickstoff aufgeschnitten und zwar jeweils in longitudinaler Richtung L und transversaler Richtung T. (Zur Definition der longitudinalen L und transversalen Raumrichtung T siehe Koordinatenkreuz in Figur 4 oben.) Die aufgeschnittenen Proben wurden auf Halterungen fixiert und in einer Magnetronanlage (Emitech, UK) besputtert. Die so präparierten Proben wurden in einem Elektronenmikroskop mit Schottky-Strahler (LEO 1550 VP, Deutschland) untersucht.

Die SEM Bilder der Vergleichsprobe (Figur 4c und f) zeigen, dass die Poren eine in alle Raumrichtungen gleichmäßige Ausdehnung und dementsprechend keine einheitliche Orientierung aufweisen. Vielmehr gleichen die Poren in longitudinaler Schnittrichtung (Figur 4c) und transversaler Schnittrichtung (Figur 4f) einander und weisen eine in alle Raumrichtungen annähernd symmetrische runde Form auf.

Auf der anderen Seite zeigen die Poren der nach dem erfindungsgemäßen Verfahren aufgeschäumten Proben dramatische Unterschiede in longitudinaler Schnittrichtung (Figur 4b) und transversaler Schnittrichtung (Figur 4c). Während der transversale Schnitt in Figur 4 c einen annähernd runden Porenquerschnitt ähnlich wie bei der isotropen Probe zeigt, ist im longitudinalen Schnitt in Figur 4b eine deutlich längliche Porengeometrie mit im Wesentlichen gleich ausgerichteten Poren in longitudinaler Raumrichtung L erkennbar. Das bedeutet, dass die Poren der erfindungsgemäßen anisotropen Probe entlang der Hauptströmungsrichtung L des entweichenden Kohlenstoffdioxids bei der Aufschäumung eine wesentlich längere Ausdehnung haben als in der orthogonal zur Hauptströmungsrichtung L verlaufenden Ebene. Die Porengeometrie lässt sich annäherungsweise als zylindrisch beschreiben, wobei ein mittlerer Porendurchmesser von 150 µm und eine mittlere Porenlänge von 900 µm berechnet wurden (SIS Soft Imaging Solutions, Scandium Software, Olympus GmbH). Dies entspricht einem Längen-Breitenverhältnis von 6.

### 4. Mechanische Eigenschaften der unprogrammierten Proben

Für die Untersuchung der mechanischen Eigenschaften der unprogrammierten Probe wurden quaderförmige Probenkörper mit Seitenlängen von 12 mm aus den anisotropen und isotropen PDLCL-Schäumen ausgeschnitten. Mit diesen Proben wurden Kompressions-Spannungs-Messungen jeweils in longitudinaler und transversaler Richtung bei 25°C mit einem Zugspannungsgerät (Instron 3345) mit einer 500 N-Lastzelle durchgeführt. Die Messungen wurden mit einer konstanten Kompressionsrate von 3,0 mm/min unter Erfassung der Spannung ausgeführt. Jede Messung wurde mit fünf Proben wiederholt und die Mittelwerte gebildet.

Das Ergebnis ist in Figur 5 gezeigt, in der die gemessene Spannung in Abhängigkeit von der prozentualen Kompression der Probe dargestellt ist. Bei allen Messungen deformieren im anfänglichen elastischen Regime die Zellwände der Poren, bevor im weiteren Verlauf das Material eine plastische Verformung erfährt. Schließlich fallen bei weiterer Zunahme der Kompression die Zellen zusammen, sodass die Proben sich wie ein kompaktes Material verhalten und die Spannung sehr stark ansteigt. Im Falle der isotropen Vergleichsprobe (Figur 5b) zeigen die Spannungsverläufe bei Kompression in longitudinaler Richtung L und in transversaler Richtung T annähernd identische Verläufe. Im Gegensatz hierzu sind für die erfindungsgemäße anisotrope Probe (Figur 5a) deutliche Unterschiede zwischen den Messungen in beiden Raumrichtungen festzustellen. So zeigt sich, dass die Materialspannung in longitudinaler Richtung L über den gesamten Kompressionsbereich größer ist als in transversaler Richtung T. Insbesondere bei niedriger Kompressionsbelastung bis etwa 10 % zeigt der longitudinale Spannungsverlauf einen deutlich steileren Anstieg als der transversale (s. Ausschnittsvergrößerung in Figur 5a). Das bedeutet, dass bei Kompression in longitudinaler Richtung die Poren einen höheren mechanischen Widerstand bieten als bei Kompression in transversaler Richtung. Diese Messungen beweisen, dass der erfindungsgemäße Schaum bereits ohne thermomechanischer Programmierung anisotrope mechanische Eigenschaften aufweist.

### 5. Thermomechanische Programmierung der Schaumproben

Quaderförmige Probenkörper der PDLCL-Schäume mit Seitenlängen von 12 mm wurden einem dreistufigen thermomechanischen Prozess zur Programmierung einer temporären Form unterzogen, um das Formgedächtnisverhalten zu untersuchen: Zunächst wurde der (in seiner herstellungsbedingten permanenten Form vorliegende) Probenkörper auf eine Temperatur T_{high} = 70°C erwärmt und bei dieser Temperatur für 5 min getempert, um eine eventuelle thermische oder mechanische Vorgeschichte des Schaums zu löschen. In einem zweiten Schritt wurde der Schaumkörper auf die Programmierungstemperatur T_{prog} = 60°C, also oberhalb der Schmelztemperatur von PCL (Tₘ = 48°C) und unterhalb der Schmelztemperatur von PPDL (Tₘ = 82°C), gebracht und bei dieser Temperatur mit einer vorbestimmten Kompressionsgrad (εₘ = 50, 65 oder 80 %) deformiert. Anschließend wurde unter Aufrechterhaltung der Kompressionskraft der Schaum mit einer schnellen Abkühlrate von 30 °C/min auf T_{low} = 0°C abgekühlt und für 10 min bei dieser Temperatur gehalten.

Diese Prozedur wurde sowohl mit den erfindungsgemäßen anisotropen Schäumen als auch mit den isotropen Vergleichsschäumen durchgeführt, wobei die Kompression jeweils in die longitudinale sowie in die transversale Richtung erfolgte.

### 6. Formgedächtnisverhalten

Die gemäß dem unter 5. Verfahren programmierten Proben wurden in einem mit einer thermostatisierbaren Kammer ausgestatteten Zugspannungsmessgerät (Zwick Z1.0 und Zwick 005) thermomechanischen Zugspannungsuntersuchungen unterworfen. Hierfür wurden die Proben mit einer Aufheizrate von 1°C/min auf eine Temperatur T_{high} = 70°C aufgeheizt, um den Formenübergang von der programmierten temporären Form (komprimierte Form) in die permanente Form auszulösen. Die Programmierungsschritte und die anschließende Rückstellung wurden dabei zyklisch wiederholt. Zwei unterschiedliche Messmodi wurden bei der Rückstellung angewandt: die lastfreie Rückstellung, bei der die Dehnung der Probe in Abhängigkeit der Temperatur erfasst wurde, sowie die Rückstellung unter einer konstanten Dehnung von 50%, bei der die Druckspannung in Abhängigkeit der Temperatur gemessen wurde. Im Falle der spannungsfreien Rückstellung ergibt sich die charakteristische Schalttemperatur T_{switch} aus dem Wendepunkt der Dehnungs-Temperatur-Kurve, während bei der Rückstellung unter Last die Spannungs-Temperatur-Kurve ein charakteristisches Maximum bei T_{σ,max} aufweist.

Die Dehnungsverläufe der lastfreien Rückstellung sind in Figur 6 für die anisotrope Probe (a) sowie für die isotrope Probe (b) dargestellt. Es ist erkennbar, dass die anisotrope Probe ein unterschiedliches Rückstellungsverhalten in die longitudinale und transversale Richtung aufweist. Insbesondere zeigt sie in longitudinaler Richtung eine Wiederherstellungsrate Rᵣ von etwa 88 %, die um etwa 20 % höher ist als in transversaler Richtung. Hingegen weist die isotrope Probe erwartungsgemäß ein für beide Raumrichtungen L und T übereinstimmendes Rückstellverhalten auf mit einer Wiederherstellungsrate Rᵣ von etwa 84 %. Die durch von der Polymerzusammensetzung abhängige und durch den Schmelzübergang von PCL bestimmte charakteristische Schalttemperatur T_{switch} ist für alle Proben und Raumrichtungen identisch.

Der unter Last rückstellende anisotrope Schaum in Figur 7 zeigt sowohl nach longitudinaler als auch transversaler Kompression gut definierte charakteristische Maxima T_{σ,max} bei etwa 48°C. Auch hier erreicht die nach longitudinaler Programmierung erhaltene Temperatur-Spannungs-Kurve einen höheren Wert für εₘ als nach transversaler Programmierung.

Figur 8 schließlich zeigt Temperatur-Dehnungs-Kurven der lastfreien Rückstellung eines anisotropen Schaums nach longitudinaler Kompression bei abwechselnden Programmierungstemperaturen (T_{prog} = 40°C und 60°C). Dieses Experiment belegt, dass wie auch bei anderen (isotropen) Formgedächtnispolymeren die Schalttemperatur durch die bei der Programmierung angewandte Temperatur gesteuert werden kann. Insbesondere weist der Schaum nach Deformierung bei 40°C eine um etwa 10 K niedrigere Schalttemperatur T_{switch} als nach Programmierung bei 60°C auf. Dieses Phänomen wird auch als Temperaturgedächtniseffekt oder Temperature-Memory-Effect (TME) bezeichnet.

### BEZUGSZEICHEN

- 10: Schäumungsvorrichtung
- 12: Druckbehälter
- 14: Prozessbehälter
- 16: Thermostat
- 18: Treibmittelvorratsbehälter
- 20: Wärmetauscher
- 22: Pumpe-Wärmetauscher-Modul
- 24: Kryostat
- 26: Absperrorgan
- 28: Absperrorgan
- 30: Belüftungsventil
- 32: regelbares Entspannungsmodul
- 34: Drucktransmitter

## Patentansprüche

1. Schaumstoffformkörper mit anisotropen, thermisch induzierbaren Formgedächtniseigenschaften, umfassend zumindest ein Formgedächtnispolymer, welches nach einer mechanischen oder thermomechanischen Programmierung in der Lage ist, temperaturinduziert zumindest einen Formenübergang von einer temporären Form in eine permanente Form zu vollziehen, **dadurch gekennzeichnet, dass** das nicht programmierte Formgedächtnispolymer eine Schaumstruktur mit asymmetrischen, im Wesentlichen in einer gemeinsamen, ersten Raumrichtung (L) ausgerichteten Poren aufweist, wobei ein Verhältnis einer mittleren Porendimension in der ersten Raumrichtung (L) zu einer mittleren Porendimension in einer zweiten, orthogonal zu dieser verlaufenden Raumrichtung (T) zumindest 2 beträgt.

2. Schaumstoffformkörper nach Anspruch 1, wobei ein Verhältnis einer mittleren Porendimension in der ersten Raumrichtung (L) zu einer mittleren Porendimension in einer zweiten, orthogonal zu dieser verlaufenden Raumrichtung (T) zumindest 3 beträgt, vorzugsweise zumindest 4, besonders bevorzugt zumindest 5.

3. Schaumstoffformkörper nach einem der vorhergehenden Ansprüche, wobei eine Dichte des Schaumstoffformkörpers im Bereich von 0,01 bis 0,30 g/cm³, insbesondere von 0,02 bis 0,20 g/cm³, vorzugsweise von 0,07 bis 0,13 g/cm³, liegt.

4. Schaumstoffformkörper nach einem der vorhergehenden Ansprüche, wobei das Formgedächtnispolymer ein physikalisch oder kovalent vernetztes Polymernetzwerk umfasst, das zumindest ein Schaltsegment aufweist, das ausgewählt ist aus der Gruppe der Polyester, insbesondere Poly(ε-caprolacton); Polyether, Polyurethane, insbesondere Polyurethan; Polyimide, Polyetherimide, Polyacrylate, Polymethacrylate, Polyvinyle, Polystyrole, Polyoxymethyle, Poly(para-dioxanon).

5. Schaumstoffformkörper nach einem der vorhergehenden Ansprüche, wobei das Formgedächtnispolymer hydrolytisch spaltbare Gruppen enthält, die insbesondere ausgewählt sind aus der Gruppe bestehend aus Glycoliden, Lactiden, Diglycoliden, Dilactiden, Polyanhydriden und Polyorthoester.

6. Artikel enthaltend oder bestehend aus einem Schaumstoffformkörper nach einem der Ansprüche 1 bis 5.

7. Artikel nach Anspruch 6, wobei der Artikel ein Gegenstand zur Anwendung im medizinischen, pharmazeutischen oder biochemischen Bereich ist.

8. Verfahren zur Herstellung eines Schaumstoffformkörpers mit anisotropen, thermisch induzierbaren Formgedächtniseigenschaften nach einem der Ansprüche 1 bis 5 mit den Schritten
(a) Herstellen einer im Wesentlichen homogenen Mischung einer Schmelze eines Formgedächtnispolymers, welches nach einer Programmierung in der Lage ist, temperaturinduziert zumindest einen Formenübergang von einer temporären Form in eine permanente Form zu vollziehen, und eines in einem ersten Zustand vorliegenden Treibmittels oder einer chemischen Vorstufe eines solchen,
(b) Überführen und Freisetzen des Treibmittels in einen zweiten, gasförmigen Zustand derart, dass das Formgedächtnispolymer und das Treibmittel entmischen, wobei Prozessparameter der Freisetzung und eine Geometrie eines Prozessbehälters, in welchem die Freisetzung des Treibmittels erfolgt, so gewählt sind, dass das freigesetzte gasförmige Treibmittel im Wesentlichen entlang einer vorbestimmten Hauptströmungsrichtung aus dem Formgedächtnispolymer entweicht, so dass das Formgedächtnispolymer eine Schaumstruktur mit asymmetrischen, im Wesentlichen in einer gemeinsamen ersten, der Hauptströmungsrichtung entsprechenden longitudinalen Raumrichtung (L) ausgerichteten Poren ausbildet, wobei die Geometrie des Prozessbehälters so gewählt ist, dass dieser in die longitudinale Raumrichtung (L) ein- oder beidseitig geöffnet und in den anderen Raumrichtungen geschlossen ist und dass die Mischung des Formgedächtnispolymers und des Treibmittels eine Säule im Prozessbehälter einnimmt, die in der longitudinalen Richtung (L) eine größere Ausdehnung aufweist als in einer orthogonal zu dieser verlaufenden transversalen Richtung (T).

9. Verfahren nach Anspruch 8, wobei, in einem weiteren Schritt (c) der Schaumstoffformkörper für seine thermomechanischen Programmierung bei einer Materialtemperatur oberhalb einer Schalttemperatur des Formgedächtnispolymers unter Anwendung eines Formenzwangs deformiert wird und unter Aufrechterhaltung des Formenzwangs auf eine Temperatur unterhalb der Schalttemperatur abgekühlt wird.

10. Verfahren nach einem der Ansprüche 8 oder 9, wobei als Treibmittel ein physikalisches Treibmittel verwendet wird, das unter der Temperatur und dem Druck des Schrittes (a) flüssig, fest oder in einem superkritischen Zustand vorliegt, und seine Freisetzung in Schritt (b) dadurch erfolgt, dass die Temperatur und/oder der Druck so verändert wird, dass das Treibmittel in den gasförmigen Zustand übergeht.

11. Verfahren nach Anspruch 10, dass als Treibmittel Kohlendioxid verwendet wird, das in Schritt (a) in superkritischem Zustand vorliegt und durch Druckabsenkung in Schritt (b) in den gasförmigen Zustand übergeht.

12. Verfahren nach einem der Ansprüche 10 oder 11, wobei in Schritt (b) das Treibmittel durch Druckabsenkung freigesetzt wird, wobei die Druckabsenkung insbesondere mit einer Rate von mindestens 10 bar/s, vorzugsweise von mindestens 15 bar/s, besonders bevorzugt von mindestens 20 bar/s erfolgt.

13. Verfahren nach einem der Ansprüche 8 bis 12, wobei in Schritt (a) ein im flüssigen oder superkritischen Zustand vorliegendes Treibmittel verwendet wird, in welchem ein weiterer Stoff, insbesondere ein pharmazeutischer Wirkstoff gelöst vorliegt.

## Claims

1. Molded foam body having anisotropic, thermally inducible shape memory properties, comprising at least one shape memory polymer which, after a mechanical or thermomechanical programming, is able to execute at least one shape transition temperature-induced from a temporary shape to a permanent shape, **characterized in that** the unprogrammed shape memory polymer exhibits a foam structure having asymmetric pores, oriented substantially in a common, first spatial direction (L), wherein a ratio of an average pore dimension in the first spatial direction (L) to an average pore dimension in a second spatial direction (T) extending orthogonally thereto is at least 2.

2. Molded foam body according to claim 1, wherein a ratio of an average pore dimension in the first spatial direction (L) to an average pore dimension in a second spatial direction (T) extending orthogonally thereto is at least 3, preferably at least 4, especially preferably at least 5.

3. Molded foam body according to any one of the preceding claims, wherein a density of the molded foam body is located within the range of 0.01 to 0.30 g/cm³, particularly 0.02 to 0.20 g/ cm³, preferably 0.07 to 0.13 g/cm³.

4. Molded foam body according to any one of the preceding claims, wherein the shape memory polymer is a physically or covalently crosslinked polymer network which comprises at least one switching segment that is selected from the group consisting of polyesters, especially poly(ε-caprolactone); polyethers, polyurethanes, especially polyurethane; polyimides, polyetherimides, polyacrylates, polymethacrylates, polyvinyls, polystyrenes, polyoxymethyls, poly(para-dioxanone).

5. Molded foam body according to any one of the preceding claims, wherein the shape memory polymer includes hydrolytically cleavable groups which are especially selected from the group consisting of glycolides, lactides, diglycolides, dilactides, polyanhydrides and polyorthoesters.

6. Article comprising or consisting of a molded foam body according to any one of claims 1 to 5.

7. Article according to claim 6, wherein the article is an object for use in the medical, pharmaceutical or biochemical field.

8. Method for manufacturing a molded foam body having anisotropic, thermally inducible shape memory properties according to any one of claims 1 to 5 having the steps
(a) manufacturing a substantially homogeneous mixture of a melt of a shape memory polymer which, after a programming, temperature-induced, is able to execute at least one shape transition from a temporary shape to a permanent shape, and of a propellant or a chemical precursor of such, the propellant or the precursor being present in a first state,
(b) transferring and releasing the propellant into a second, gaseous state in such a way that the shape memory polymer and the propellant separate, wherein process parameters of the release and a geometry of a process container, in which the release of the propellant takes place, are selected such that the released gaseous propellant escapes from the shape memory polymer substantially along a predetermined main flow direction, so that the shape memory polymer forms a foam structure having asymmetric pores, oriented substantially in a common, first longitudinal spatial direction (L) corresponding to the main flow direction, wherein the geometry of the process container is selected so that this is open on one or both sides in the longitudinal spatial direction (L) and is closed in the other spatial directions and so that the mixture of the shape memory polymer and the propellant occupies a column in the process container, which column in the longitudinal direction (L) has a greater extent than in a transverse direction (T) extending orthogonally thereto.

9. Method according to claim 8, wherein in a further step (c) the molded foam body, for its thermomechanical programming, is deformed at a material temperature above a switching temperature of the shape memory polymer using a deformation constraint and is cooled at a temperature below the switching temperature while maintaining the deformation constraint.

10. Method according to any one of claims 8 or 9, wherein, as the propellant, a physical propellant is used which at the temperature and the pressure of step (a) exists in a liquid, solid or supercritical state, and its release in step (b) is effected by the temperature and/or the pressure being altered such that the propellant passes over into the gaseous state.

11. Method according to claim 10, that as the propellant carbon dioxide is used which is present in step (a) in a supercritical state and by reducing the pressure in step (b) passes over into the gaseous state.

12. Method according to any one of claims 10 or 11, wherein in step (b) the propellant is released by reducing the pressure, wherein the pressure is reduced especially at a rate of at least 10 bar/s, preferably of at least 15 bar/s, especially preferably of at least 20 bar/s.

13. Method according to any one of claims 8 to 12, wherein in step (a) a propellant existing in a liquid or supercritical state is used, in which a further substance, especially a pharmaceutical active substance, is dissolved.

## Revendications

1. Corps moulé en mousse ayant des propriétés anisotropes et thermo-inductibles de mémoire de forme, comprenant au moins un polymère à mémoire de forme, lequel est en mesure, après une programmation mécanique ou thermomécanique, d'accomplir par induction de chaleur au moins une transition formelle d'une forme temporaire à une forme permanente, **caractérisé en ce que** le polymère à mémoire de forme non programmé présente une structure de mousse à portes asymétriques, orientés sensiblement dans une première direction spatiale (L) commune, un rapport entre une grandeur de pore moyenne dans la première direction spatiale (L) et une grandeur de pore moyenne dans une deuxième direction spatiale (T), orthogonale à la première, étant égal ou supérieur à 2.

2. Corps moulé en mousse selon la revendication 1, où un rapport entre une grandeur de pore moyenne dans la première direction spatiale et une grandeur de pore moyenne dans une deuxième direction spatiale (T), orthogonale à la première, est égal ou supérieur à 3, avantageusement égal ou supérieur à 4, préférentiellement égal ou supérieur à 5.

3. Corps moulé en mousse selon l'une des revendications précédentes, où une densité du corps moulé en mousse est comprise entre 0,01 et 0,30 g/cm³, avantageusement entre 0,02 et 0,20 g/cm³, préférentiellement entre 0,07 et 0,13 g/cm³.

4. Corps moulé en mousse selon l'une des revendications précédentes, où le polymère à mémoire de forme comporte un polymère réticulé physiquement ou par liaison covalente, lequel comprend au moins un segment de commutation sélectionné dans le groupe des polyesters, en particulier poly(ε-caprolactone) ; polyéther, polyuréthane, en particulier polyuréthane ; polyimides, polyétherimides, polyacrylates, polyméthacrylates, polyvinyls, polystyrènes, polyoxyméthyles, poly(para-dioxanone).

5. Corps moulé en mousse selon l'une des revendications précédentes, où le polymère à mémoire de forme comprend des groupes séparables par hydrolyse, lesquels sont en particulier sélectionnés dans le groupe comprenant des glycolides, lactides, diglycolides, dilactides, polyanhydrides et des polyorthoesters.

6. Article contenant un corps moulé en mousse selon l'une des revendications 1 à 5, ou constitué de celui-ci.

7. Article selon la revendication 6, où ledit article est un objet destiné à une application dans les domaines médical, pharmaceutique ou biochimique.

8. Procédé de fabrication d'un corps moulé en mousse ayant des propriétés anisotropes et thermo-inductibles de mémoire de forme selon l'une des revendications 1 à 5, comprenant les étapes suivantes :
(a) fabrication d'un mélange sensiblement homogène d'un polymère à mémoire de forme fondu, lequel est en mesure, après une programmation, d'accomplir par induction de chaleur au moins une transition formelle d'une forme temporaire à une forme permanente, et d'un agent moussant présenté dans un premier état ou d'un précurseur chimique d'un tel agent moussant,
(b) passage et libération de l'agent moussant vers un deuxième état gazeux de manière à dissocier le polymère à mémoire de forme et l'agent moussant, les paramètres de processus de la libération et une géométrie d'un récipient de processus où a lieu la libération de l'agent moussant étant sélectionnés de telle manière que l'agent moussant gazeux est libéré du polymère à mémoire de forme sensiblement dans une direction d'écoulement principale définie, si bien que le polymère à mémoire de forme forme une structure de mousse à portes asymétriques, orientés sensiblement dans une première direction spatiale (L) longitudinale commune correspondant à la direction d'écoulement principale, la géométrie du récipient de processus étant sélectionnée de telle manière que celui-ci soit ouvert sur un ou sur deux côtés dans la direction spatiale longitudinale (L) et fermé dans l'autre direction spatiale, et que le mélange du polymère à mémoire de forme et de l'agent moussant occupe une colonne du récipient de processus présentant dans la direction longitudinale (L) une extension supérieure à une direction transversale (T) orthogonale à ladite direction.

9. Procédé selon la revendication 8, où, lors d'une autre étape (c), le corps moulé en mousse est déformé pour sa programmation thermomécanique à une température de matériau supérieure à une température de commutation du polymère à mémoire de forme par application d'une contrainte de forme, et est refroidi à une température inférieure à la température de commutation en maintenant la contrainte de forme.

10. Procédé selon la revendication 8 ou la revendication 9, où un agent moussant physique est utilisé comme agent moussant, lequel est présenté à l'état liquide, solide ou dans un état super-critique à la température et sous la pression de l'étape (a), et en ce que sa libération à l'étape (b) est obtenue en ce que la température et/ou la pression sont modifiées de manière à faire passer l'agent moussant à l'état gazeux.

11. Procédé selon la revendication 10, où un dioxyde de carbone est utilisé comme agent moussant, lequel est présenté dans un état super-critique lors de l'étape (a) et passe à l'état gazeux par diminution de pression lors de l'étape (b).

12. Procédé selon la revendication 10 ou la revendication 11, où l'agent moussant est libéré par diminution de pression lors de l'étape (b), la diminution de pression étant effectuée en particulier avec une vitesse égale ou supérieure à 10 bar/s, avantageusement égale ou supérieure à 15 bar/s, préférentiellement égale ou supérieure à 20 bar/s.

13. Procédé selon l'une des revendications 8 à 12, où un agent moussant présenté dans un état liquide ou super-critique est utilisé lors de l'étape (a), lequel contient une autre substance dissoute, en particulier une substance active pharmaceutique.
